# EUROPEAN PATENT APPLICATION

(11) **EP 3 338 842 A1**
(43) Date of publication of application: **27.06.2018**
(21) Application number: 17150542.3
(22) Date of filing: 06.01.2017
(51) Int. Cl.: A61M 15/00, B05B 12/00, G01F 1/74

(54) **NEBULIZER FOR LAMINAR FLOW INHALATION OF AN AEROSOL**

(30) Priority: 22.12.2016 EP 16206173
(71) Applicant: Schulz, Bernhard, 10117 Berlin (DE)
(72) Inventor: Schulz, Bernhard, 10117 Berlin (DE)
(74) Representative: Schulz Junghans Patentanwälte PartGmbB

(57) **Abstract**

The invention relates to an inhaler (1) for providing a laminar flow of an aerosol for inhalation comprising at least the following components, a reservoir (2) comprising aerosol particles (21), a mouthpiece (3), wherein the mouthpiece (3) is fluidically connectable or connected to the reservoir (2), and wherein the mouthpiece (3) is designed for oral inhalation of the aerosol by a user of the inhaler (1), wherein the inhaler (1) comprises a registration means (4) that is configured to determine a fluidic parameter of an aerosol, when the aerosol is flowing through the mouthpiece (3). Furthermore the invention relates to a system comprising the inhaler (1) and a computer (100) as well as a computer program for use with the inhaler (1).

## Description

### Specification

The invention relates to an inhaler for controlled inhalation, a system comprising an inhaler for controlled inhalation and a computer program for monitoring the inhalation process.

Inhalers or nebulizers for inhaling pharmaceutical compounds or pharmaceutical compositions (in the following summarized under the term "pharmaceutical compound" only) such as cortisone or β2 adrenergic receptor agonists that are frequently used for asthma treatment are known from the state of the art. The pharmaceutical compound is provided by the inhalers as an aerosol, wherein the aerosol comprises the pharmaceutical compound as a powder or as an aerosol fluid. Inhalers and nebulizers come in many different forms and shapes, wherein either metered-dose inhalers (MDIs), which aerosolize the drug, or dry powder inhalers (DPIs), which dispense powder which can be inhaled are most commonly used. The terms "nebulizer" and "inhaler" are used synonymously in this application.

Most inhalers provide a mechanism that is configured for expelling the aerosol powder particularly from a pressurized reservoir in order to deliver the pharmaceutical compounds to the site of action, namely the bronchial system of the user.

It is thought that a faster ejection speed of the aerosol correlates with a better deposition efficiency of the pharmaceutical compound in the lower bronchial system. This delivery method however, has a number of drawbacks. The most prominent drawback is that the pharmaceutical compound is deposited also in the upper throat and mouth of the person. This might lead to unwanted side effects such as mycosis in the throat which is a common during topical cortisone treatment.

Furthermore, the relationship between the expelled dose of pharmaceutical compound and the effective dose, i.e. the amount of pharmaceutical compound that is indeed delivered to the site of action in the pulmonary system is prone to inhalation practice, user anatomy and other factors. Therefore, it is almost impossible to determine an effectively delivered dose of the specific pharmaceutical compound. Inhalers that don't comprise such an expellant mechanism but rely on suction provided by the user when inhaling the pharmaceutical compound are known from the state of the art (e.g. Easyhaler from Orion Pharma).

However, also these inhalers do not solve the problem of a controlled provision of the pharmaceutical compound to the pulmonary system, as delivery and deposition efficiency of the pharmaceutical compound depend on the individual inhalation practice.

Therefore, the problem underlying the present invention is to provide a device allowing the controlled provision of a pharmaceutical compound to the pulmonary system.

This problem is solved by an inhaler, particularly a dry powder inhaler, having the features of claim 1, a system having the features of claim 13, a computer program having the features of claim 15. Preferred embodiments are stated in the sub claims and the description. The problem according to the invention is furthermore solved by a method for laminar inhalation as well as by a method for estimating a fluidic parameter.

### Inhaler

According to claim 1, a particularly handheld inhaler for providing a particularly controllable, particularly laminar flow of an aerosol powder comprises at least the following components:
- a reservoir comprising aerosol powder or a receiving portion for receiving a reservoir comprising aerosol particles,
- a mouthpiece, wherein the mouthpiece is fluidically connectable or connected to the reservoir, and wherein the mouthpiece is designed for oral inhalation of the aerosol by a user of the inhaler, wherein the inhaler comprises a registration means that is configured to determine a fluidic parameter of an aerosol, when the aerosol is flowing through the mouthpiece.

The inhaler according to the invention solves advantageously the problem according to the invention, as it provides a registration means that is suited for a controlled provision of a specific dose of a pharmaceutical compound to the desired site of action of the pulmonary system.

As the registration means is configured to determine a fluidic parameter, it provides the basis for a controlled provision of the aerosol. The fluidic parameter can subsequently be evaluated in order to train the inhalation practice of a person using the inhaler.

The mouthpiece of the inhaler is either connected but at least fluidically connectable to the reservoir such that the aerosol powder can be inhaled through the mouthpiece.

The Inhaler can be configured to provide an exchangeable reservoir, comprising the aerosol powder. Such an inhaler is configured to receive said reservoir with a receiving portion. In the following it is assumed that the inhaler is in a state comprising a reservoir, whether it is received by the receiving portion or whether it is incorporated in the inhaler is of no importance for the invention, but solely a different embodiment that can be chosen accordingly.

Furthermore, the inhaler according to the invention is configured such that an aerosol flow through the mouthpiece is particularly only generated by a pressure difference applied by a user, particularly by inhalation through to the mouthpiece. Thus, the inhaler according to the invention particularly provides the aerosol powder, however the intake of the aerosol powder is particularly only facilitated by inhalation through the mouthpiece.

The inhaler is particularly not configured to expel the aerosol powder.

Advantageously, the registration means is determining a fluidic parameter upon use of the inhaler, wherein the fluidic parameter is particularly associated to the inhalation strength applied by the person. Therefore it is possible to control the intake of the aerosol powder by the person, by monitoring the fluidic parameter.

The registration means particularly comprises components that are arranged on the inhaler and that are exposed to a portion of the mouthpiece such that a flow of the aerosol powder can be quantified with regard to the fluidic parameter. The fluidic parameter is particularly one of: a hydrodynamic, an aerodynamic, a hydrostatic, or an aerostatic parameter of a flowing aerosol powder.

The aerosol reservoir is particularly exchangeably arranged or arrangeable on the inhaler, such that an empty reservoir can be replaced by a new or a different reservoir.

The mouthpiece comprises at least a first channel that connects the reservoir with a first opening of the mouthpiece for the mouth, such that inhalation through the first opening of the mouthpiece becomes possible.

### Registration means is arranged in the mouthpiece

According to another embodiment of the invention, the registration means is arranged at least partially in the mouthpiece, wherein the registration means is arranged such that particularly a sensor of the registration means is exposed to a hollow portion of the mouthpiece, particularly wherein the sensor of the registration means or the registration means is arranged in the aerosol flow or a separate channel of the mouthpiece leading to and particularly fluidically connected to the first opening of the mouthpiece.

### Registration means is arranged in a tube

According to another embodiment of the invention, the registration means is arranged in a particularly flexible tube, that can be placed with a first opening of the flexible tube next to the first opening of the mouthpiece, such that when a person inhales using the inhaler according to the invention, also an airflow is generated in the flexible tube, wherein the registration means is arranged such in the tube, that it can determine the fluidic parameter.

This embodiment provides advantageously a protection of the registration means from the aerosol powder, as the aerosol flow is flowing through the mouthpiece, wherein the registration means registers an airflow devoid of the aerosol, as it is arranged in a separate tube. The airflow in the tube and the aerosol flow in the mouthpiece can be considered being equivalent, such that the determined fluidic parameter of the airflow is equivalent to the fluidic parameter of the aerosol flow as well.

### Second opening in the mouthpiece

According to another embodiment of the invention, the mouthpiece of the inhaler comprises a second opening that is arranged such on the mouthpiece that additional air from the surrounding of the inhaler can flow in the mouthpiece particularly towards the first opening, when suction is applied to first opening of the mouthpiece, i.e. when the user inhales.

This embodiment is particularly advantageous as the registration means can be arranged in a particularly separate (from a channel where the aerosol flows towards the first opening) channel that connects said second opening with the mouthpiece. According to this embodiment, the registration means is protected from the aerosol and the aerosol flow, but is still capable to determine the fluidic parameter reliably. Furthermore, the registration means does not induce any turbulences in the aerosol flow caused be e.g. protrusions of the registration means reaching in the aerosol flow.

The registration means arranged in the channel connecting the second opening with the interior of the mouthpiece, i.e. the aerosol flow channel of the mouthpiece, will experience an airflow or will be able to detect sounds from inhalation or an airflow, and thus would be capable of determining the fluidic parameter.

The channel connecting the second opening with the mouthpiece can also extend further to the first opening of the mouthpiece and thus form a completely separate channel in the inhaler. The first opening in this embodiment is particularly divided (e.g. by a wall) in two segments, each comprising an output of the inhaler (one for the aerosol flow, and the other solely an air flow).

### Fluidic parameter

According to another embodiment of the invention, the fluidic parameter comprises or is at least one of:
- a volume flow of the aerosol,
- a mass flow of the aerosol,
- a flow rate of the aerosol through the mouthpiece,
- a pressure in the mouthpiece, and/or
- a pressure difference of the flowing aerosol in the mouthpiece with respect to a surrounding atmospheric pressure.

The listed fluidic parameters are each well-suited to determine a flow of the aerosol powder such that the flow of the aerosol can be evaluated with regard to a deposition efficiency at the site of action in the pulmonary system. The evaluation can be based on the degree of the flow laminarity, i.e. whether the aerosol flow is laminar, substantially laminar or turbulent inside the mouthpiece of the inhaler. Alternatively the evaluation with regard to deposition efficiency can be based on empirical data for the specific fluidic parameter or values derived from test measurements or test series.

In case the fluidic parameter comprises or is a pressure difference, said pressure difference is determined along the flow of the aerosol powder in the mouthpiece, i.e. the pressure difference is particularly determined between a portion inside the mouthpiece, particularly inside the first channel of the mouthpiece and the surrounding (outside of the inhaler) atmospheric pressure .

Depending on the kind of fluidic parameter that is to be determined, the registration means of the inhaler comprises specific components that are configured to determine the specific fluidic parameter.

For example, if the fluidic parameter is a pressure difference, the registration means is or comprises a pressure sensor that is configured to determine a pressure difference with regard to the surrounding atmospheric pressure. In case the fluidic parameter is a flow rate, the registration means comprises particularly a flow meter that is configured for determining a flow rate of the aerosol powder current through the mouthpiece.

### Registration means

According to another embodiment of the invention, the registration means is or comprises at least one of the following devices:
- a pressure sensor that is particularly configured to determine a pressure difference with regard to the surrounding atmospheric pressure,
- a flow meter that is particularly configured for determining a flow rate of the aerosol powder current through the mouthpiece,
- a sound sensor that is particularly configured to determine a sound from the flowing aerosol or the person inhaling the aerosol.

### Modularity

According to another embodiment of the invention, the inhaler comprises a reservoir portion and a module portion, wherein the module portion is releasably attachable to the reservoir portion of the inhaler, wherein the module portion can be released by a releasing means, wherein said releasing means particularly comprises a releasable catch means, wherein the reservoir or the receiving portion is arranged on the reservoir portion of the inhaler, wherein particularly the registration means is arranged on the module portion.

This embodiment takes advantageously into account that parts or components of the inhaler might be reusable and/or of a certain value, such that a disposal of these parts or components is economically unfavourable. By dividing the inhaler in two portions that are detachable from each other, it is possible to reuse particularly the components that are connected to the registration means, or components that comprise an electronic circuit, compounds that comprise noble earth, or energy sources such as batteries, as they can be arranged on the module portion.

The releasing means can comprise a releasable 'click'-fastener or a turning-fastener. The mouthpiece is preferably arranged on the reservoir portion.

### Electronic circuit

According to another embodiment of the invention, the inhaler comprises an electronic circuit, particularly an integrated circuit, more particularly a microcontroller, more particularly a microprocessor, wherein the electronic circuit is arranged for retrieving and evaluating the fluidic parameter determined by the registration means, wherein the electric circuit is particularly arranged on the module portion.

The advantage of such an electronic circuit is that the fluidic parameter can be processed on the inhaler and data comprising information about the fluidic parameter can be generated on the inhaler, such that no additional external device is required. This embodiment is the basis for an all-integrated approach facilitating a controlled inhalation of the aerosol powder by a person. Furthermore it is particularly advantageous to arrange the electronic circuit on the module portion in case the inhaler comprises such a portion.

For the purpose of receiving the determined fluidic parameter and performing various operations with that information, it is advantageous if the electronic circuit is or comprises a microcontroller.

The electronic circuit is particularly connected to the registration means.

### Maximum and Minimum value

According to another embodiment of the invention, the electronic circuit, particularly the microcontroller, is configured to determine whether
- the determined fluidic parameter reaches or exceeds a predefined maximum value, and/or
- the fluidic dynamic parameter reaches or falls below a predefined minimum value.

For this purpose it is advantageous if the electronic circuit comprises a memory storage.

This embodiment is particularly advantageous, as with the determination of whether the fluidic parameter is within certain limits, it can be concluded of whether a resulting aerosol powder flow is in or close to the laminar flow regime. A person using the inhaler that is able to determine whether a provided flow of an aerosol powder reaches or exceeds a maximum value can be advantageously informed about his or her inhalation practice.

Furthermore, monitoring an aerosol powder flow can be achieved by comparing the provided aerosol powder flow characterized by the fluidic parameter with a maximum value and/or a minimum value for said fluidic parameter.

### Mechanical indicator

According to another embodiment of the invention, the inhaler comprises a mechanical means that is configured to determine whether
- the determined fluidic parameter reaches or exceeds a predefined maximum value, and/or
- the fluidic dynamic parameter reaches or falls below a predefined minimum value.

Such an inhaler provides the same functionality as an inhaler with an electronic circuit for evaluating the fluidic parameter, but without the need of an energy source. In another embodiment of the invention the mechanical means is further configured to particularly visually signal to a user, whether the fluidic parameter exceeds the maximum predefined value or whether the fluidic parameter falls below the minimum predefined value, or even if the determined fluidic parameter is within the interval between the minimum and maximum predefined value.

For this purpose the mechanical means might comprise a coloured component with a plurality of distinct colours, wherein a specific colour of the component becomes visible to the user depending on the determined fluidic parameter.

### On-board signalling means

According to another embodiment of the invention, the inhaler comprises a signalling means, that is configured to output a signal, when the determined fluidic parameter reaches or exceeds the predefined maximum value and/or wherein the signalling means of the inhaler is configured to output a signal, when the determined fluidic parameter reaches or is below the predefined minimum value and/or wherein the signalling means is configured to output a signal, wherein the signal is a function of the actual determined fluidic parameter, particularly wherein the signalling means is arranged on the module portion of the inhaler.

An inhaler according to this embodiment is configured to determine and evaluate the fluidic parameter as well as to indicate to the user or a person in the surrounding, such as a doctor, whether the users inhalation practice leads to a fluidic parameter that exceeds the predefined maximum value or falls below the predefined minimum value.

With this indication, a controlled provision of the aerosol powder flow can be achieved, as the person using the nebuliser is enabled to adapt his or her inhalation practice depending on the particularly instantaneously issued signal of the inhaler. According to this embodiment, it is also conceived that the signalling means issues a signal wherein the signal is a function of the actual determined fluidic parameter. For example, if the signal is an acoustic signal, the pitch of the signal can be used as an indicator of the value of the determined fluidic parameter. The goal for a person using the inhaler could then be, to generate a signal with a certain, constant pitch indicating that the inhalation practice, for example the strength of inhalation is just right for the desired provision of the aerosol powder.

This embodiment is particularly advantageous as a signal might be used for training of the inhalation practice of a person, such that the person is particularly able to inhale with just the right strength and duration, such that particularly an essentially laminar flow inside the mouthpiece of the inhaler is achieved.

### Visual, acoustic, or tactile signal

According to another embodiment of the invention, the signalling means is configured to issue a signal that is an acoustic signal, a visual signal and/or a tactile signal. Depending on the environment or certain special needs of the person using the inhaler, it might be advantageous to issue an acoustic signal, for example if the person using the inhaler has impaired vision.

In case a more discreet use of the inhaler is intended, it might prove advantageous to issue a visual signal, for example by displaying the value of the fluidic parameter or - more intuitive - to display the value of the fluidic parameter using a colour code or look-up-table. For example, the colour blue can be used to indicate a too low fluidic parameter that is the determined fluidic parameter is below the minimum value, the colour red can be issued in order to indicate that the determined fluidic parameter exceeds the maximum value, and the colour green can be used to indicate that the fluidic parameter is within the interval between the minimum and maximum value. Also, a tactile signal could be used in order to inform the user of the inhaler about his actual inhalation practice with regard to the fluidic parameter. A tactile signal can be issued by a vibration or different vibration patterns.

Depending on what kind of signal is to be issued by the inhaler, the corresponding signalling means has to be chosen accordingly.

The signalling means therefore comprises for example a loudspeaker, a display, a colour indicator, or a vibration motor.

### Communication unit

According to another embodiment of the invention, the inhaler comprises a communication unit that is configured to send data from the inhaler to an external computer or to receive data from the external computer, wherein the communication unit is particularly arranged for retrieving data from the electronic circuit of the inhaler, wherein the data particularly comprises information about the actual determined fluidic parameter, wherein the communication unit is particularly arranged on the module portion.

The communication unit might comprise a radiofrequency transmitter and receiver. The communication unit is advantageously comprised by the module portion and enables a communication with an external device like a computer, a mobile phone a smart phone tablets.

The term "computer" refers to an electronic device that comprises at least a microcontroller and that is configured to execute a software program that is loaded on or streamed to the computer.

The communication unit therefore can make use of an existing computer infrastructure of the person. Thus, tasks of evaluation of the determined fluidic parameter or indication of an actual fluidic parameter can be outsourced to said computer infrastructure - reducing the number of components on the inhaler and thus reducing the cost of manufacture.

Furthermore, it allows greater flexibility in terms of conveying information to the user about his or her inhalation practice.

### Energy source

According to another embodiment of the invention, the inhaler comprises an energy source for the electronic circuit, wherein the energy source is arranged on the module portion and wherein the energy source is particularly a battery or an accumulator.

### Bluetooth

According to another embodiment of the invention, the communication unit is configured to send the signal as radio waves within the framework of wireless personal area networks (WPANs), particularly in the frequency range of 2.400 to 2.485 GHz, commonly known as "Bluetooth".

### Limiting means

According to another embodiment of the invention, the inhaler comprises a limiting means, wherein the limiting means is configured to limit the fluidic parameter to a predefined maximum value, wherein the limiting means is particularly arranged on the module portion.

This embodiment is particularly advantageous, as it prevents the aerosol powder flow of exceeding a certain flow speed or flowrate. Therefore a misuse of the inhaler is prevented.

### Definition of maximum value

According to another embodiment of the invention, the maximum predefined value is
- 100%, particularly 50%, more particularly 25% higher than,
- identical to, or
- 75%, particularly 50%, more particularly 25%, lower than
the fluidic parameter at which the flow of the aerosol through the mouthpiece switches from a laminar flow regime to a turbulent flow regime of the aerosol.

There are several ways to characterise the laminar flow regime. A common way to characterise a laminar flow regime is by estimating the so-called Reynolds number. Fluid flowing through a certain geometry (e.g. a tube) with a certain viscosity and a certain flow speed will give rise to a specific Reynolds number. Above a critical Reynolds number the flow will become turbulent, below the critical Reynolds number flow will remain substantially laminar. Thus, the critical Reynolds number can be used to determine the maximum predefined value.

Alternatively, the maximum predefined value is chosen preferably such that the majority of the aerosol powder, once inhaled, reaches the site of action in the bronchial system, when inhaled with an associated fluidic parameter below the predefined maximum value. As discussed above, it is also possible to determine the maximum predefined value by test measurements.

### Definition minimum value

According to another embodiment of the invention, the minimum predefined value is 75%, particularly 80%, more particularly 90% below the fluidic parameter at which the flow of the aerosol through the mouthpiece switches from a laminar flow to a turbulent flow.

The line between a laminar and a turbulent flow regime can be drawn by the Reynolds number as explained above. Alternatively, the minimum predefined value can be chosen based on empirical tests.

### Memory storage

According to another embodiment of the invention, the maximum and/or minimum predefined value is stored electronically, wherein the maximum predefined value is particularly stored on a memory storage of the inhaler, particularly on the module portion, wherein the maximum predefined value is accessible by the electronic circuit. By storing the maximum predefined value electronically the value can be adjusted for example when the inhaler geometry is changed.

### USB

According to another embodiment of the invention, the inhaler comprises means for establishing a particularly wireless personal area network with an external computer. Said means particularly comprise a USB connection, a Firewire connection, more particularly an IrDA connection, wherein said means is configured to receive and/or send data via the personal area network.

This embodiment allows the programming of the inhaler particularly the electronic circuit more particularly the microcontroller or microprocessor of the inhaler, wherein particularly the maximum predefined value can be provided to the inhaler via said means.

### Sound sensor

According to another embodiment of the invention the registration means is or comprises a sound sensor, the sound sensor particularly being configured to determine the fluidic parameter from the recorded sound.

### Microphone

According to another aspect of the invention the sound sensor is a microphone.

### Sound sensor is arranged in mouthpiece

According to another embodiment of the invention the sound sensor is arranged in the mouthpiece of the inhaler.

### Energy source

According to another embodiment of the invention the inhaler comprises an energy source, particularly a battery, an rechargeable battery or accumulator, a thermoelectric generator or a solar cell, wherein the energy source is particularly releasably housed in the inhaler.

### System comprising an inhaler and a computer

The problem according to the invention is furthermore solved by a system comprising an inhaler according to the invention and an external computer, wherein the inhaler comprises a communication unit that is configured to send and/or receive particularly repeatedly, intermittently or continuously data comprising or relating to the determined fluidic parameter from the inhaler to the computer.

By providing means to the inhaler that enables the communication of the inhaler with an external computer, for example a mobile device such as a smart phone, data comprising the fluidic parameter can be displayed, or signalled via the external computer in order to inform the user of the inhaler about his inhalation practice.

As many people possess a computer, such as for example a smart phone, the system according to the invention solves the problem by externalizing some components, such as for example a display, a loudspeaker, or a more powerful microprocessor, to an external computer.

An external computer is not configured to be comprised by the inhaler and is furthermore particularly not comprised by the inhaler.

The computer in turn might comprise software that is configured to evaluate the received data with regard to the fluidic parameter, the frequency of use of the inhaler, and/or other data related to the inhaler or its use, which is an advantageous way to control and monitor a fluidic parameter of an aerosol powder flow.

The data might comprise the determined fluidic parameter or relate to the fluidic parameter. It is for example possible to derive the total amount of aerosol dispensed by the inhaler by logging the determined fluidic parameter over time. Furthermore it is possible to estimate an effective dosage of a pharmaceutical compound that is supposedly delivered to the site of action of the user of the inhaler, by determining the amount of aerosol dispensed based on the fluidic parameter and then weight this amount by a predefined factor that particularly takes into account losses and concentration of the pharmaceutical compound.

### Mobile phone application

According to another embodiment of the invention, the computer is designed to evaluate the data and to issue a signal depending on the determined fluidic parameter, wherein the signal
- is issued when the fluidic parameter reaches or exceeds a predefined maximum value,
- is issued when the fluidic dynamic parameter falls below a predefined minimum value, and/or
- is repeatedly or continuously issued and wherein the signal is correlated to the determined fluidic parameter, wherein the signal changes with the absolute or relative value of the determined fluidic parameter.

This embodiment allows for precise control of a fluidic parameter and its associated aerosol powder flow.

The signal issued by the computer can be a visual, an acoustic and/or a tactile signal as described above. Furthermore, depending on the signal, a software program running on the computer might execute additional tasks, such as accessing databases related to the use of a pharmaceutical compound. The computer program can also be configured store the received data in order to build a usage history.

### Performance parameter

According to another embodiment of the invention, the computer is configured to particularly repeatedly, intermittently or continuously
- determine a performance parameter that comprises the acquired fluidic parameter and a target fluidic parameter, or a deviation, a difference or a quotient thereof,
- put out, particularly display the performance parameter particularly such that a user of the inhaler is instructed to change or retain his inhaling practice such that the fluidic parameter matches the target fluidic parameter.

The target fluidic parameter can be chosen such that a substantially laminar flow is achieved in the mouthpiece of the inhaler.

### Dosage monitoring

According to another embodiment of the invention, the computer or a computer program running on the computer is configured to determine a difference between a predefined target amount of the aerosol to be dispensed by the inhaler or inhaled by the user and the determined amount of aerosol that has been dispensed by the inhaler or inhaled by the user and to indicate or display how much aerosol is to be dispensed by the inhaler or how much more aerosol has to be inhaled by the user until the predefined target amount is reached, and particularly wherein the computer or the computer program running on the computer is configured to issue a signal to the user, when the determined amount of aerosol reaches or surpasses the predefined target amount of aerosol.

This embodiment allows advantageously a dosage monitoring that is particularly helpful for long time use of the inhaler. Based on the data of the dosage monitoring a health status of the person can be established.

### Telemedicine System

According to another embodiment of the invention, the inhaler and the external computer are comprised by a tele-evaluation system for a centralized remote evaluation of the data from the inhaler, particularly by medical personnel, wherein the tele-evaluation system comprises means for remote medical interaction with a user of the inhaler, wherein the data is sent to the centralized remote evaluation by a telecommunication means, particularly for communication via a radio network, via an optical network, via the internet, or via another suitable computer network.

The system according to the invention can be incorporated in a tele-evaluation system also known as telemedicine system. Thus, the inhaler and the use of the inhaler can be monitored by medical personnel, which at the same time can further instruct a user of the inhaler how to use the inhaler properly, while the medical personnel is not at the same site than the user. The tele-evaluation system comprises the telecommunication means that is configured to connect to the centralised remote evaluation by the common means of a radio network such as a mobile phone network, e.g. 3G, 4G or 5G, or any other common networks that provide a suitable telemedicine infrastructure.

Alternatively or simultaneously the computer of the user can for example be connected to a computer network. The centralized remote evaluation site might comprise a computer as well that is configured to communicate with the computer of the user via the network. This way instructions and information can be shared between the medical personnel and the user.

### Computer program

The problem according to the invention is also solved by a computer program for controlling and monitoring an aerosol powder flow through the mouthpiece of an inhaler, particularly for use with an inhaler according to the invention or a system according to the invention, wherein the computer program comprises computer program code that is configured to execute the following steps, when the computer program is executed or loaded on a computer:
- issue a signal when the determined fluidic parameter exceeds a predefined maximum value,
- issue a signal when the fluidic parameter falls below a predefined minimum value,
- repeatedly, intermittently or continuously issue a signal, wherein the signal is correlated to the determined fluidic parameter, wherein the signal changes with the absolute or relative value of the determined fluidic parameter.

Such a computer program is particularly useful and advantageous for use with an inhaler according to the invention, as it is configured to provide a feedback to the user of the inhaler about his inhalation practice, particularly whether the determined fluidic parameter is within the limits of the minimum and maximum predefined value for the fluidic parameter, or which value the determined fluidic parameter is currently adopting.

Such a particularly instantaneous feedback relating to the use of an inhaler is well-suited to also provide training with regard to the inhalation practice of a user. Furthermore, such a computer program can be configured to provide further instructions about the frequency and duration of use of the inhaler.

### Computer program with a target fluidic parameter

According to another embodiment of the invention, the computer program is executes the steps of
- determining a target fluidic parameter, particularly by fetching said target fluidic parameter from a database or an electronic storage medium,
- acquiring particularly repeatedly, intermittently or continuously a fluidic parameter of an inhaler,
- determining a performance parameter, that comprises or is derived from the acquired fluidic parameter and the target fluidic parameter, wherein the performance parameter is particularly a deviation, a difference or a quotient of the acquired fluidic parameter and the target fluidic parameter,
- issuing, outputting, displaying or externalizing said performance parameter such that a user of the inhaler is instructed or informed particularly how to change or retain his inhaling practice such that the fluidic parameter matches the target fluidic parameter.

### User-related data

According to another embodiment of the invention, the computer program further comprises the steps of:
- acquiring user-related data, wherein the user-related data comprise an age, a sex and/or a weight of a user of the inhaler,
- determining the target fluidic parameter from the user related-data.

As for example a dosage of the aerosol or the inhalation ability can depend on the age, sex or the weight of a user of the inhaler this embodiment is particularly advantageous, as it takes into account these factors which might influence a good medication practice.

The user-related data might be provided by the user or by medical personnel. These user-related data are advantageously provided to the computer program before first use of the inhaler.

### Computer program application

According to another embodiment of the invention, the computer program further comprises the steps of:
- determining from the determined fluidic parameter an amount of aerosol that has been dispensed by the inhaler or that has been inhaled by the user,
- particularly electronically storing or electronically submitting the determined amount to a memory storage, that can be a remote database server, or a storage arranged on the inhaler or a memory storage of a personal computer, such a as smart phone.
- particularly electronically storing or electronically submitting usage information, wherein the usage information comprises a time of use of the inhaler, and an indicator of the used aerosol.

This embodiment provides the advantage that not only the fluidic parameter is determined, but also a total dosage that can be evaluated for example for medical purposes. This way, medical personnel might be enabled to adapt or change the pharmaceutical compound used for treatment of the user of the inhaler by evaluating said information.

### Encryption

According to one embodiment of the invention data that are determined, stored, send, received or submitted is encrypted for protection of personal data.

### Dosage instructions

According to another embodiment of the invention, the computer program comprises the steps of:
- determining a difference between a predefined target amount of the aerosol to be dispensed by the inhaler or inhaled by the user and the determined amount of aerosol that has been dispensed by the inhaler or inhaled by the user, and
- indicating or displaying how much more the inhaler has to dispense or the user has to inhale until the predefined target amount is reached, and
- particularly wherein the computer program issues a signal to the user, when the determined amount of aerosol reaches or surpasses the predefined target amount of aerosol.

The predefined target amount can be a daily dose that should to be inhaled by the user. The computer program according to this embodiment particularly monitors the dispensed amount of aerosol with regard to a predefined target amount.

### Reminder function

According to another embodiment of the invention, the computer program issues a signal to the user after a predefined time of the last use of the inhaler.

This embodiment is particularly useful, when a pharmaceutical compound should be inhaled repeatedly and with a certain frequency. For example it is possible to remind a user of the inhaler in predefined intervals that can be programmed beforehand, to inhale the pharmaceutical compound using the inhaler.

The signal can be an acoustic, a visual or a tactile signal. Also the computer program might issue said signal in form of an electronic message such as email, a text message or by use of a suitable messenger service.

### Health status

According to another embodiment of the invention, the computer program is provided with a user input, wherein the user provides information about the actual health status and pain.

By logging the use of the inhaler and monitoring the health status and pain of the user, potential side effects or the efficacy of the pharmaceutical compound can be detected or monitored.

### Computer-Telemedicine

According to another embodiment of the invention, the computer program comprises computer program code that is configured and designed to communicate with a tele-evaluation system, particularly with a telemedicine server.

### Method for laminar flow inhalation

The problem according to the invention is furthermore solved by a method for inhaling an aerosol from an inhaler according to the invention comprising a mouthpiece, characterized in that the inhalation strength through the mouthpiece of the inhaler is adjusted such that the resulting aerosol flow or an associated fluidic parameter is within a laminar flow regime or below a maximum predefined value.

### Method for estimating a fluidic flow parameter

Furthermore the problem according to the invention is solved by a method for inhalation of an aerosol from an inhaler, particularly an inhaler according to the invention, comprising the steps of:
- Providing an aerosol powder in an inhaler that can be inhaled when a person provides an airflow to the inhaler,
- Providing an aerosol flow through the mouthpiece of the inhaler by inhaling the aerosol powder through the mouthpiece,
- Estimating a fluidic parameter with the registration means.

The such estimated fluidic flow parameter can be used for a controlled and accurate provision of an aerosol powder.

### Flow estimation with sound

According to another embodiment of the invention the method further comprises the steps of
- Determining a sound from the flowing aerosol powder and/or the person providing the aerosol flow by inhalation particularly with a sound sensor of the inhaler,
- Estimating the fluidic parameter from the determined sound.

Estimating the fluidic parameter with a sound sensor such as for example a microphone, offers a reliable way of estimating the fluidic parameter as sound patterns can be compared to a database comparably efficient.

### Comparison with pre-recorded sounds

According to another embodiment of the invention the fluidic parameter is determined by comparison of the determined sound and a predefined sound, particularly a pre-recorded sound.

This embodiment allows for the precise estimation of a fluidic parameter such as for example flow speed, as the sound generated by the inhalation process can be related to a flow speed or a pressure difference.

Thus, by predefining a sound or a sound pattern, said predefined sound can be compared for example by means of correlation algorithms or other techniques for estimating a similarly of two signals. The results can then be used for estimating whether the such determined fluidic parameter is within predefined limits, such as defined for example by the maximum value and/or the minimum value.

The predefined sound can be for example be recorded under supervision of trained personnel. When under these training conditions the fluidic parameter is just right, e.g. flow speed or pressure difference is such that the flow is essentially laminar, the corresponding sound is saved for example to a memory storage of the inhaler and can be compared to sounds that are recorded at different times, particularly without supervision.

If the recorded sound is sufficiently similar to the pre-recorded sound the inhalation technique of the person is just right.

In case the similarity is not high enough, a feed back in terms of a signal can be given to the user of the inhalator.

There are several well-known techniques to estimate and establish a robust similarity measure for such an application, such as for example correlation algorithms, or state of the art supervised or unsupervised learning algorithms, or established example pattern matching algorithms.

The comparison of the recorded and predefined sound can be facilitated with an external computer, such as a mobile phone, or on a suitable device, such as a microprocessor on the inhaler.

Further features and advantages of the invention shall be described by means of a detailed description of embodiments with reference to the figures. It is shown in:
- Fig. 1: a schematic cross-section through an inhaler according to the invention; and
- Fig. 2: a system comprising an inhaler and a mobile phone;

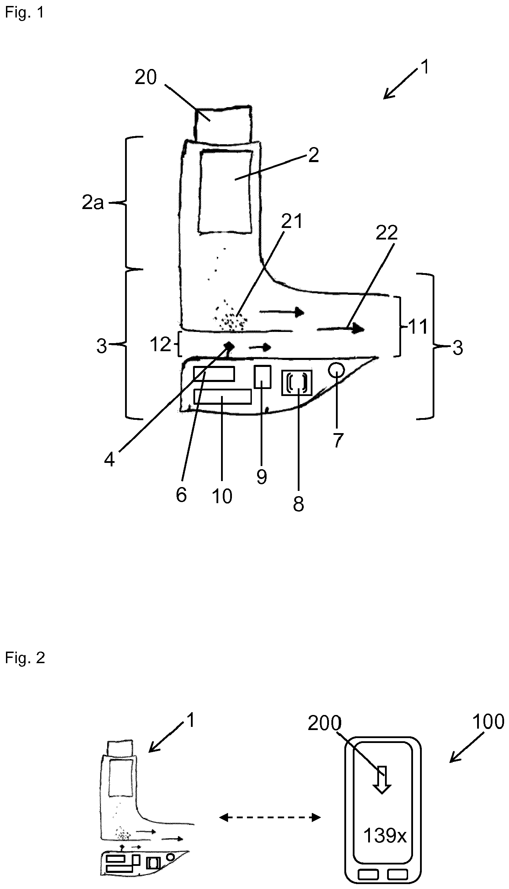

Fig. 1 shows a cross-section of an inhaler 1. The inhaler 1 comprises a receiving portion 2a for the aerosol powder reservoir 2 with an aerosol powder reservoir 2.

The receiving portion 2a is connected to a mouthpiece 3 through which the aerosol can be inhaled.

The mouthpiece 3 has a first opening 11, through which the aerosol flow 22 is inhaled. A second opening 12 is arranged at the back of the mouthpiece 3. The second opening 12 is fluidically connected to the first opening 11 by a channel that separates the aerosol flow 22 from air streaming through the second opening 12 towards the first opening 11. In the channel a registration means 4 in form of a pressure sensor is arranged. The pressure sensor is configured to measure a fluidic parameter, in this example a pressure difference, when a user is inhaling the aerosol through the mouthpiece 3. As the pressure sensor is arranged in the channel, it is advantageously protected from the aerosol flow 22.

The mouthpiece 3 furthermore comprises an energy source 10 in form of a battery, an electronic circuit 6 in form of a microcontroller, a memory storage device 9 in form of a flash storage device, a communication unit 8 in form of a bluetooth transmitter/receiver unit and a signalling means 7 in form of a loudspeaker.

The inhaler 1 furthermore comprises a powder release button 20 that can be pressed in order to release a defined amount of aerosol powder 21 into the mouthpiece 3. The fluidic parameter determined from pressure sensor is transmitted to the microcontroller 6 and processed in order to determine whether the estimated fluidic parameter is within the limits of a maximum value of the flow parameter that is stored on the microcontroller 6 or the memory storage 9, and a minimum value of the flow parameter that is also stored in the microcontroller 6 or the memory storage 9.

If the determined fluidic parameter is above the predefined maximum value of the flow parameter, a signal is sent to the signalling means 7, which in turn issues an acoustic signal indicating to the user that the suction applied to the mouthpiece 3 is too strong and the aerosol flow is not ideal for effective inhalation.

The frequency or another distinguishable characteristic of the acoustic signal can be proportional to the amount the determined fluidic parameter.

Alternatively or simultaneously the microcontroller can send the determined fluidic parameter to the communication unit 8 that in turn sends data comprising the fluidic parameter to an external device such as a computer or a server.

The battery 10 provides the required electricity to the components of the inhaler 1. The battery 10 is stored exchangeably in the lower part of the mouthpiece 3.

Fig. 2 shows a schematic system comprising an inhaler 1 and an external computer 100. In the example shown, the external computer 100 is a mobile phone that comprises a computer program that is configured to display the estimated fluidic parameter or an indicator related to the fluidic parameter such as an arrow, indicating whether the user should apply less suction (arrow points downwards) to the mouthpiece or more (arrow points upwards). The estimated fluidic parameter is sent (indicated by the broken arrow) form the inhaler 1 to the mobile phone.

The computer program can furthermore be configured to display the number of uses 139x of the inhaler 1 on the display of the mobile phone.

In the following an exemplary background of the invention is given and some optional, non-limiting features of the inhaler, the system or the computer program (also referred to as "app") are disclosed.

Asthma and COPD are among the most common diseases worldwide, affecting approximately 10% of the world's population.

The therapy of both diseases is based essentially on the local application of active substances in the bronchial system. However, worldwide it is not standardized how to use inhaled medicines (powders or sprays). Each manufacturer develops own, in each case differently shaped and applicable inhalers, which are usually to be used with a strong inspiratory flow.

The high internal resistance of the devices requires a high airflow velocity to ensure complete release of the active substance from the inhaler. Passing the mouthpiece of the device, the airflow will become turbulent and the powder or spray is inevitably swirled. The passage of the active substances through the oral cavity and especially the vocal chords is nearly impossible and the deposition of the drugs in the deep bronchial system succeeds only to a small extent. Significant proportions of active substances (e.g. cortisone and bronchodilators) remain in the oral cavity and cause side effects such as hoarseness, oral thrush, restlessness, heart palpitations, dizziness, and sleep disturbances.

The suboptimal application of these actually very effective drugs, leads to a suboptimal therapeutic success with high morbidity (cough, dyspnoea, serious infections, increasing disability and mortality (according to WHO, COPD will be the third most common cause of death by 2020).

A different, for most of the patient easy implementable, but so far not established inhalation-technique can lead to a significant improvement in patient care. At present this different inhalation-technique is practicable with only a few inhalers that allow a slow and approximately laminar inspiratory flow.

The different drug-application leads to a significantly improved symptom-control and simultaneously to a significant reduction of emergency procedures, morbidity and illness dependent missed days. This implicates the opportunity to a significant reduction of outpatient and inpatient costs.

This treatment strategy has led to a dramatic reduction in the number of emergency treatments, and a significant reduction in drug costs of about 30% according to the evaluation of the health insurance system, while maintaining a high patient satisfaction. Through patient training and the consistent implementation of the therapy no emergency-i.v. therapy had to be performed for about 5 years and no patients have been admitted to a hospital due to acute respiratory symptoms.

The inhaler or some embodiments according to the invention features the following advantages:
- controlled slow and almost laminar flow inhalation,
- Integration of an inspiratory flow measurement,
- Bluetooth interface and connection to a smartphone,
- Control of inhalation quality and efficiency via an app,
- Entering the current health status, subjective well-being feeling into the app,
- Correlation of patient subjective well-being with inhalation quality,
- Telemedicine-based patient care.

## Claims

1. Inhaler (1), for providing a flow of an aerosol (22) for inhalation comprising at least the following components:
- a reservoir (2) comprising aerosol powder or a receiving portion (2a) for receiving a reservoir (2) comprising aerosol powder,
- a mouthpiece (3), wherein the mouthpiece (3) is fluidically connectable or connected to the reservoir (2) or the receiving portion (2a), and wherein the mouthpiece (3) is designed for oral inhalation of the aerosol by a user of the inhaler (1),
**characterized in that**
the inhaler (1) comprises a registration means (4) that is configured to determine a fluidic parameter of an aerosol, when the aerosol is flowing through the mouthpiece (3).

2. Inhaler according to claim 1, **characterized in that** the fluidic parameter comprises or is at least one of:
- a volume flow of the aerosol,
- a mass flow of the aerosol,
- a flow rate of the aerosol through the mouthpiece,
- a pressure in the mouthpiece, and/or
- a pressure difference of a flowing aerosol in the mouthpiece.

3. Inhaler according to claim 1 or 2, wherein the registration means (4) is or comprises at least one of the following devices:
- a pressure sensor,
- a flow meter,
- a sound sensor, the sound sensor being configured to record the fluidic parameter.

4. Inhaler according to one of the preceding claims, wherein the inhaler (1) comprises a reservoir portion and a module portion, wherein the module portion is releasably attachable to the reservoir portion of the inhaler (1), wherein the module portion can be released by a releasing means, wherein said releasing means particularly comprises a releasable catch means, wherein the reservoir (2) or the receiving portion (2a) is arranged on the reservoir portion of the inhaler (1), wherein particularly the registration means (4) is arranged on the module portion.

5. Inhaler according to one of the preceding claims, wherein the inhaler (1) comprises an electronic circuit (6), particularly a microprocessor, wherein the electronic circuit is arranged for retrieving and evaluating the fluidic parameter determined by the registration means (4), wherein the electric circuit is particularly arranged on the module portion.

6. Inhaler according to claim 5, wherein the electronic circuit (4) is arranged for determining whether
- the determined fluidic parameter reaches or exceeds a predefined maximum value, and/or
- the fluidic dynamic parameter reaches or falls below a predefined minimum value.

7. Inhaler according to claim 6, wherein the inhaler (1) comprises a signaling means (7), that is configured to issue a signal, when the determined fluidic parameter reaches or exceeds the predefined maximum value and/or wherein the signaling means (4) of the inhaler (1) is configured to issue a signal, when the determined fluidic parameter reaches or is below the predefined minimum value and/or wherein the signaling means (4) is configured to issue a signal, wherein the signal is a function of the actual determined fluidic parameter, particularly wherein the signaling means is arranged on the module portion of the inhaler (1).

8. Inhaler according to claim 7, wherein the signaling means (4) is configured to issue a signal that is an acoustic signal, a visual signal and/or a tactile signal.

9. Inhaler according to one of the preceding claims, **characterized in that** the inhaler (1) comprises a communication unit (8) that is configured to send and/or receive data from the inhaler (1) to an external computer (100), wherein the communication unit (8) is particularly arranged for retrieving data from the electronic circuit (6), wherein the data particularly comprise information about the actual determined fluidic parameter, wherein the communication unit (8) is particularly arranged on the module portion.

10. Inhaler according to one of the claims 6 to 9, **characterized in that** the maximum predefined value is
- 100%, particularly 50%, more particularly 25% higher than,
- identical to, or
- 75%, particularly 50%, more particularly 25%, lower than
the fluidic parameter at which the flow (22) of the aerosol through the mouthpiece (3) switches from a laminar flow to a turbulent flow of the aerosol.

11. Inhaler according to claim 3, wherein the sound sensor is arranged in the mouthpiece (3) of the inhaler (1).

12. Inhaler according to one of the preceding claims, wherein the inhaler (1) comprises an energy source (10), particularly a battery, a rechargeable battery or an accumulator, a thermoelectric generator or a solar cell, wherein the energy source is particularly releasably housed in the inhaler (1).

13. System comprising an inhaler (1) according to one of the preceding claims and an external computer (100), wherein the inhaler (1) comprises a communication unit (8) particularly according to claim 9 that is configured to send and/or receive particularly repeatedly, intermittently or continuously data comprising or relating to the determined fluidic parameter from the inhaler (1) to the computer (100).

14. System according to claim 13, wherein the computer (100) is designed to evaluate the data and to issue a signal depending on the determined fluidic parameter, wherein the signal
- is issued when the fluidic parameter reaches or exceeds a predefined maximum value,
- is issued when the fluidic dynamic parameter falls below a predefined minimum value,
- is repeatedly or continuously issued and wherein the signal is correlated to the determined fluidic parameter, wherein the signal changes with the absolute or relative value of the determined fluidic parameter.

15. Computer program for use with an inhaler particularly according to one of the claims 1 to 12 and for use in a system particularly according to claims 13 to 14, comprising computer program code, wherein the computer program comprises computer program code that is configured to execute the following steps, when the computer program is executed or loaded on a computer:
- issue a signal when the determined fluidic parameter exceeds a predefined maximum value,
- issue a signal when the fluidic parameter falls below a predefined minimum value,
- repeatedly, intermittently or continuously issue a signal, wherein the signal is correlated to the determined fluidic parameter, wherein the signal changes with the absolute or relative value of the determined fluidic parameter.
